# EUROPEAN PATENT APPLICATION

(11) **EP 4 379 379 A1**
(43) Date of publication of application: **05.06.2024**
(21) Application number: 22849193.2
(22) Date of filing: 07.07.2022
(51) Int. Cl.: G01N 33/543

(54) **TEST CARTRIDGE**

(30) Priority: 30.07.2021 JP 2021126034
(71) Applicant: FUJIFILM Corporation, Tokyo 106-8620 (JP)
(72) Inventor: ISHII, Hiroyasu, Ashigarakami-gun, Kanagawa 258-8538 (JP)
(74) Representative: Hughes, Andrea Michelle
(86) International application number: PCT/JP2022/026930
(87) International publication number: WO 2023/008128

(57) **Abstract**

Provided is an assay cartridge capable of improving the throughput of the assay by shortening the time until the bubbles generated in the assay region disappear as compared with the prior art. The assay cartridge includes an assay strip that has, on the surface thereof, an assay region in which a color development state changes depending on whether a sample is positive or negative, an accommodating part that accommodates an amplifying liquid that amplifies the color development of the assay region, and the assay cartridge that is supplied from the accommodating part, an absorber that absorbs at least a part of the amplifying liquid which flows on the surface of the assay strip toward the assay region and has passed through the assay region.

## Description

### BACKGROUND OF THE INVENTION

### 1. Field of the Invention

The present disclosure relates to an assay cartridge.

### 2. Description of the Related Art

JP2010-71827A and JP2011-99724A describe an immunochromatographic kit for performing an assay of whether a sample is positive or negative, that is, whether or not the sample contains a test substance, using an immunochromatographic method. This immunochromatographic kit is called an assay cartridge or the like. The immunochromatographic kit described in JP2010-71827A and JP2011-99724A includes a sample, an assay strip in which a reagent such as an amplifying liquid is supplied, and a case in which the assay strip is accommodated.

The assay strip includes an assay region on which an antibody that specifically binds to an antigen, which is a test substance, is immobilized. In a case where a labeled antibody that specifically binds to an antigen is developed on an assay strip together with a sample containing the antigen, the antigen binds to the antibody immobilized on the assay region and the labeling substance is captured via the antigen. In a case where the amount of the labeling substance captured in this assay region is very small, the color development is weak and it may be determined that the sample is false negative. In the immunochromatographic method described in JP2010-71827A and JP2011-99724A, an amplifying liquid is supplied to an assay region to amplify the color development of the assay region due to the labeling substance. Whether the sample is positive or negative is determined based on the density of the assay region amplified by the amplifying liquid. An accommodating part that accommodates an amplifying liquid is provided in the case, and the amplifying liquid is supplied from the accommodating part to the assay strip and developed toward the assay region.

### SUMMARY OF THE INVENTION

The amplifying liquid may permeate the assay strip and be developed toward the assay region by the capillary force of the assay strip, or may flow on the surface of the assay strip toward the assay region. In a case where the amplifying liquid flows on the surface of the assay strip, bubbles may be generated in the amplifying liquid. The bubbles of the amplifying liquid become noise in a case where the color development state of the assay region is determined. Therefore, in order to make an accurate determination in the assay, it is necessary to wait for the disappearance of the bubbles. The amplifying liquid is retreated from the assay region with the lapse of time and the bubbles disappear, but there is a problem that in a case where a time until the bubbles disappear is long, the throughput of the assay is reduced. In a case where the amplifying liquid flows on the surface of the assay strip, a part of the amplifying liquid that has once passed through the assay region flows back toward the assay region. The back flow of the amplifying liquid causes the time for which the bubbles disappear to prolong.

The present disclosure has been made in view of the above circumstances, and an object of the present disclosure is to provide an assay cartridge capable of improving the throughput of the assay by shortening the time until the bubbles generated in the assay region disappear as compared with the prior art.

The assay cartridge of the present disclosure is an assay cartridge that is used for immunochromatographic assay, the assay cartridge comprising
an assay strip that has, on a surface, an assay region in which a color development state changes depending on whether a sample is positive or negative,
an accommodating part that accommodates an amplifying liquid which amplifies color development of the assay region, and
an absorber that absorbs at least a part of the amplifying liquid which is supplied from the accommodating part, flows on the surface of the assay strip toward the assay region, and passes through the assay region.

In the assay cartridge of the present disclosure, it is preferable that the absorber is arranged on a downstream side of the assay region in a flow direction of the amplifying liquid, and the absorber is not overlapped with the assay region in the flow direction.

In the assay cartridge of the present disclosure, in a case where a direction intersecting the flow direction of the amplifying liquid is defined as a width direction, the absorber is preferably arranged on at least one of both sides of the assay strip in the width direction. The absorber is particularly preferably arranged on both sides of the assay strip in the width direction.

In the assay cartridge of the present disclosure, at least a part of the absorber may be arranged at a position facing the surface of the assay strip.

In the assay cartridge of the present disclosure, the absorber is preferably arranged with an interval from the assay strip.

In the assay cartridge of the present disclosure, the interval between the absorber and the assay strip is preferably 0.3 mm or more and 1 mm or less.

In the assay cartridge of the present disclosure, in the assay strip, a spotting region on which the sample is spotted is preferably arranged on a downstream side of the assay region in a flow direction of the amplifying liquid.

The assay cartridge of the present disclosure preferably includes a second absorber different from the absorber described above.

The second absorber is arranged on an upstream side, in a flow direction of the amplifying liquid, of a supplying portion of the amplifying liquid from the accommodating part of the assay strip. The second absorber absorbs a sample which is developed in a direction opposite to a flow direction of the amplifying liquid and which passes through the assay region.

According to the assay cartridge of the present disclosure, the throughput of the assay can be improved by shortening the time until the bubbles generated in the assay region disappear as compared with the prior art.

### BRIEF DESCRIPTION OF THE DRAWINGS

Fig. 1 is a perspective view of an assay cartridge according to the present disclosure.
Fig. 2 is an exploded perspective view of the assay cartridge according to the present disclosure.
Fig. 3A is a partially broken side view showing a state in which a first pressing operation part of the assay cartridge according to the present disclosure is pushed in, and Fig. 3B is a partially broken side view showing a state in which the first pressing operation part and a second pressing operation part are pushed in.
Fig. 4 is a side view showing a positional relationship between an assay strip, a multifunctional member, a first amplifying liquid holding part, and a second amplifying liquid holding part, in the assay cartridge according to the present disclosure.
Fig. 5 is an explanatory diagram of an immunochromatographic method.
Fig. 6 is a plan view showing a positional relationship between an assay strip and an absorber in the assay cartridge.
Fig. 7 is a cross-sectional view taken along the line VII-VII of Fig. 6.
Fig. 8 is a plan view showing another example of the positional relationship between an assay strip and an absorber in the assay cartridge.
Fig. 9 is a plan view showing further another example of the positional relationship between an assay strip and an absorber in the assay cartridge.
Fig. 10 is a cross-sectional view taken along line X-X of Fig. 9.

### DESCRIPTION OF THE PREFERRED EMBODIMENTS

Hereinafter, an assay cartridge according to an embodiment of the present invention is described with reference to the drawings. The constituent elements indicated by the same reference numerals in the drawings mean the same constituent elements. However, unless otherwise specified in the specification, each constituent element is not limited to one, and a plurality of each constituent element may be present.

In addition, description of overlapping configurations and reference numerals in the respective drawings may be omitted. The present invention is not limited to the following embodiments, and can be implemented with appropriate modifications, such as omitting a configuration or replacing a configuration with a different configuration within the scope of the object of the present invention.

The directions indicated by the arrows X and Y, which are appropriately shown in the respective figures, are directions along the horizontal plane and are orthogonal to each other. In addition, the direction indicated by the arrow Z is a direction along the perpendicular direction (vertical direction). The directions indicated by the arrows X, Y, and Z in respective figures coincide with each other.

### <Overview of assay cartridge>

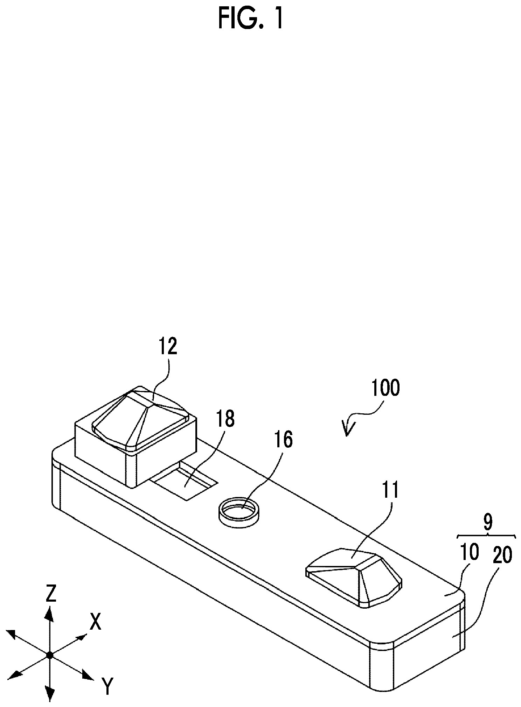

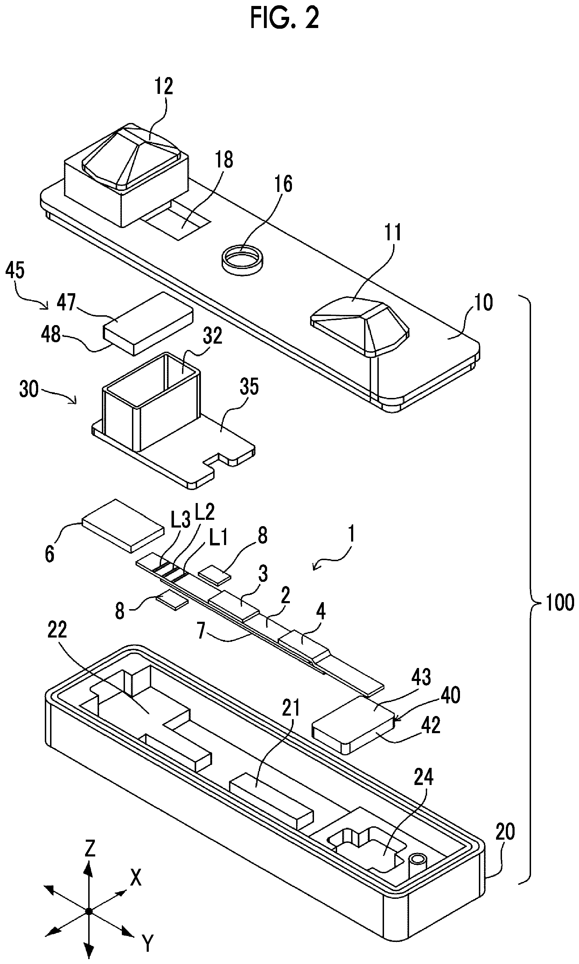

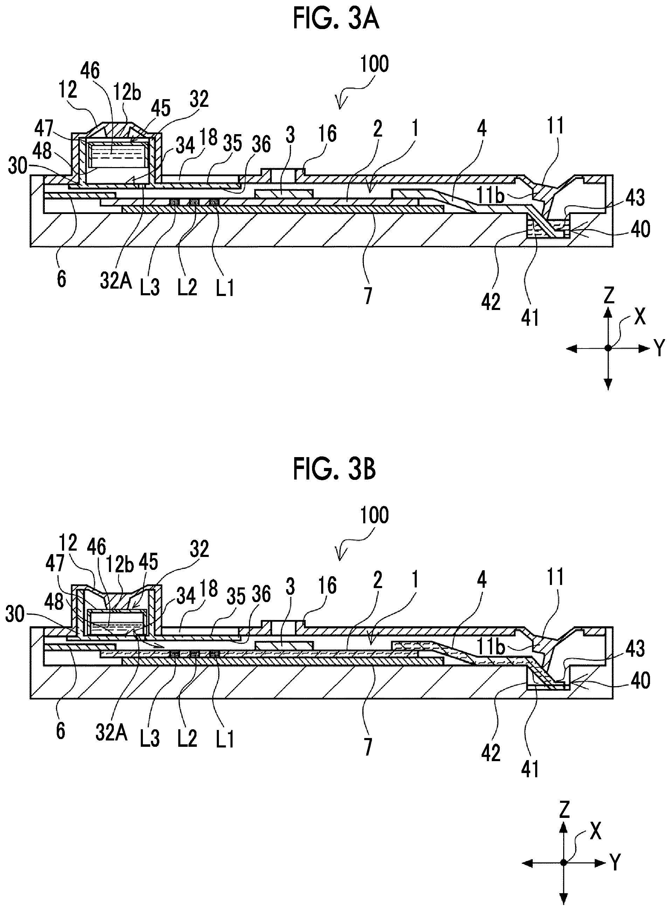

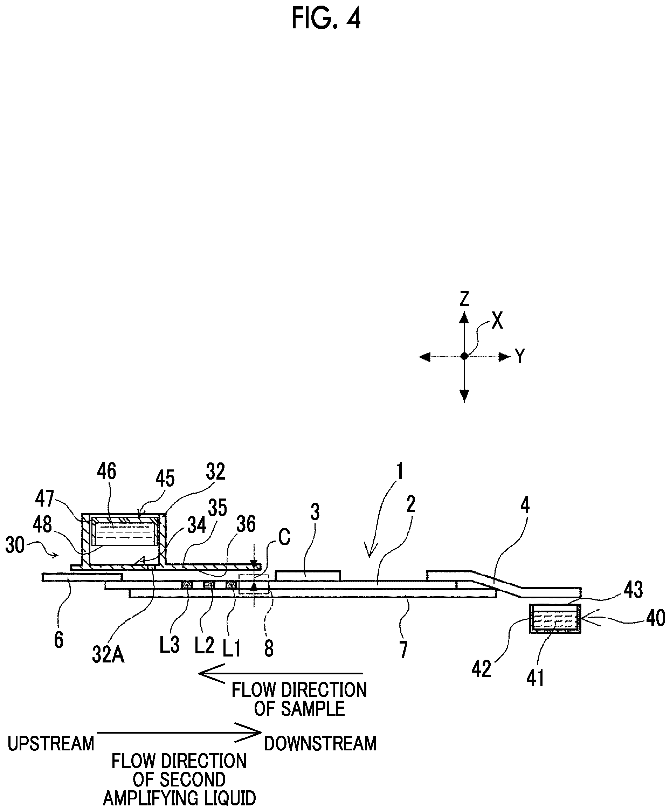

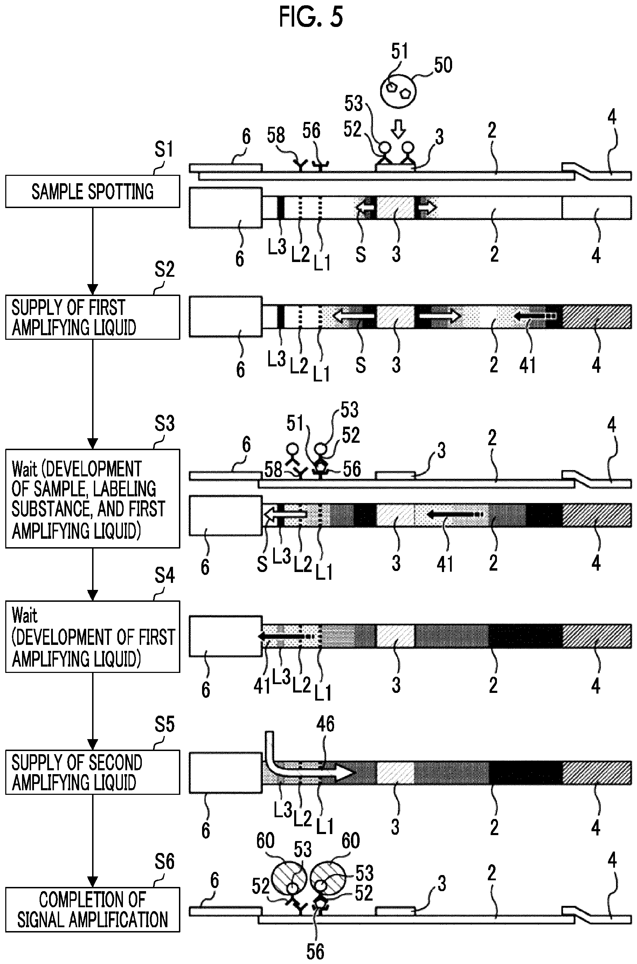

Fig. 1 is an external view of an assay cartridge 100 (Hereinafter, referred to as a cartridge 100) according to an embodiment, and Fig. 2 is an exploded perspective view of the cartridge 100. Fig. 3 is a diagram showing a state in which the first pressing operation part 11 and the second pressing operation part 12 provided in the cartridge 100 are operated. More specifically, Fig. 3A shows a state in which the first pressing operation part 11 is operated, and Fig. 3B shows a state in which the first pressing operation part 11 and the second pressing operation part 12 are operated. Fig. 4 is a diagram showing the main accommodated components in the cartridge 100. Fig. 5 is an explanatory diagram of an immunochromatographic method.

The cartridge 100 is a single-use type that is used one by one in each sample of assay target. As shown in Fig. 2, an assay strip 1 including an immunochromatographic carrier 2 (hereinafter, referred to as a carrier 2) is provided in the cartridge 100. An assay region L1 is provided in the carrier 2, and the color development state changes depending on whether or not the sample contains a test substance, that is, whether the sample is positive or negative. In addition, absorbers 8 are provided on both sides of the immunochromatographic carrier 2 in the width direction.

The "change in color development state" includes any of an aspect in which a first color different from the color of the carrier 2 changes to another second color (that is, a color change), an aspect in which the color of the carrier 2 changes to another color by developing a color different from that of the carrier 2 (that is, color development), and an aspect in which the density of the color changes (that is, a density change).

The sample is simply required to be a specimen that may contain a test substance, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, a liquid specimen containing animals and plants themselves or a dried body thereof. Examples of the test substance include an antigen, an antibody, a protein, and a low-molecular-weight compound.

The cartridge 100 has a configuration that allows a user to visually confirm whether the sample is positive or negative. Such a cartridge 100 is also referred to as an immunochromatographic assay tool, an immunochromatographic assay kit, or the like.

As shown in Fig. 1 and Fig. 2, as an example, the cartridge 100 includes a case 9 constituted of a case main body 20 and a cover member 10. The case 9 is formed of, for example, a resin material. An opening is formed in an upper part of the case main body 20, and in addition to the assay strip 1, absorbers 8, a first amplifying liquid holding part 40, a second amplifying liquid holding part 45, and the like are accommodated therein. The cover member 10 covers the opening of the case main body 20 by being attached to the opening part of the case main body 20. The case 9 has an elongated shape as a whole in accordance with the elongated shape of the assay strip 1.

In the present example, a dropping port 16, an observation window 18, a first pressing operation part 11, and a second pressing operation part 12 are provided on an upper part of the case 9 constituted of the cover member 10. Each of these parts is integrally molded with the cover member 10 as an example. The dropping port 16 is an opening for adding dropwise a sample into the inside of the case 9. A boss is vertically provided on the edge of the dropping port 16 toward the upper part.

### (Observation window)

An observation window 18 is an opening portion for observing the assay region L1 from the outside, in the present example, the size of the observation window 18 is a size such that, in addition to the assay region L1, the control region L2 and the color development region L3, which is described below, can also be observed. The user can confirm whether the sample is positive or negative by observing the color development state of the assay region L1 in the observation window 18.

### (First pressing operation part and second pressing operation part)

As shown in Fig. 2, Fig. 3A, and Fig. 3B, the first pressing operation part 11 is an operating part operated to supply the first amplifying liquid 41 in the first amplifying liquid holding part 40 to the assay strip 1. The second pressing operation part 12 is an operating part operated to supply a second amplifying liquid 46 in the second amplifying liquid holding part 45 to the assay strip 1. As described later, the first amplifying liquid 41 and the second amplifying liquid 46 are amplifying liquids for amplifying the color development in the assay region L1 in a case where the sample is positive.

As shown in Fig. 3A, in a case where a pressing force is applied to the first pressing operation part 11 by a pressing operation by a user, or the like, the first pressing operation part 11 is deformed. As shown in Fig. 2, as an example, the first pressing operation part 11 has a quadrangular pyramid shape, and in a case where a pressing force is applied from above to a region including the apex of the quadrangular pyramid, as shown in Fig. 3A, the first pressing operation part 11 is deformed such that the apex of the quadrangular pyramid sinks into the inside of the case 9. In a case where the first pressing operation part 11 sinks, the first pressing operation part 11 presses the first amplifying liquid holding part 40 inside the case 9 via the end part of the assay strip 1. By this pressing, a part of the first amplifying liquid holding part 40 breaks. The end part of the assay strip 1 enters into the first amplifying liquid holding part 40 from this broken part and is immersed in the first amplifying liquid 41. As described below, the assay strip 1 is formed of a porous material. As a result, in the assay strip 1, the first amplifying liquid 41 is sucked up by the capillary force from the portion of the assay strip 1, which is immersed in the first amplifying liquid 41. Accordingly, the first amplifying liquid 41 is supplied to the assay strip 1. The first amplifying liquid 41 supplied to the assay strip 1 is developed through the inside of the assay strip 1 by capillary action, toward the assay region L1.

In addition, the first pressing operation part 11 is deformed by pressing and then the deformed state is maintained. Accordingly, after the first pressing operation part 11 is pressed, the supply of the first amplifying liquid 41 to the assay strip 1 is continued until all the first amplifying liquid 41 is sucked up.

Similarly, as shown in Fig. 3B, in a case where a pressing force is applied to the second pressing operation part 12, the second pressing operation part 12 is deformed. As shown in Fig. 2, similarly to the first pressing operation part 11, the second pressing operation part 12 of the present example also has a quadrangular pyramid shape, and in a case where a pressing force is applied from above to a region including the apex of the quadrangular pyramid, as shown in Fig. 3B, the second pressing operation part 12 is deformed such that the apex of the quadrangular pyramid sinks into the inside of the case 9. In the second pressing operation part 12 of the present example, an abutting part 12b that abuts on the second amplifying liquid holding part 45 is provided. In a case where the second pressing operation part 12 is deformed, the abutting part 12b presses the second amplifying liquid holding part 45 inside the case 9. The second amplifying liquid holding part 45 is partially broken by a pressing force applied from the abutting part 12b. The second amplifying liquid holding part 45 is arranged at a position facing the surface of the assay strip 1 with an interval from the surface of the assay strip 1. In a case where the second amplifying liquid holding part 45 breaks, the second amplifying liquid 46 held by the second amplifying liquid holding part 45 flows out from the broken part onto the surface of the assay strip 1. Accordingly, the second amplifying liquid 46 is supplied to the assay strip 1.

The second amplifying liquid 46 supplied to the assay strip 1 flows toward the assay region L1 on the surface of the assay strip 1. Then, a part of the second amplifying liquid 46 that has reached the assay region L1 infiltrates the assay region L1. In this manner, similarly to the first amplifying liquid 41, the second amplifying liquid 46 is supplied to the assay strip 1, but unlike the amplifying liquid 41 being developed within the assay strip 1 by the capillary force, the second amplifying liquid 46 flows on the surface of the assay strip 1. Here, the second amplifying liquid 46 is an example of an "amplifying liquid" according to the technology of the present disclosure described in the scope of the claims.

In addition, similarly to the first pressing operation part 11, the second pressing operation part 12 is preferably deformed by pressing and then the deformed state is maintained. It is because in a case where the deformation of the second pressing operation part 12 is maintained even after the user releases the hand, the supply of the second amplifying liquid 46 is easier to be continued.

### (First amplifying liquid holding part)

As shown in Fig. 2, Fig. 3A, and Fig. 3B, the case main body 20 accommodates the assay strip 1 including the carrier 2 along the longitudinal direction. As shown in Fig. 3A, Fig. 3B, and Fig. 4, in the case main body 20, the first amplifying liquid holding part 40 is arranged on one end part (right end part side in Fig. 4) in the longitudinal direction. In the case main body 20, in a portion where the first amplifying liquid holding part 40 is arranged, the first accommodating part 24 having a recess-shaped in accordance with the shape of the first amplifying liquid holding part 40 is formed. One end part of the assay strip 1 is arranged above the first amplifying liquid holding part 40 in a state of being accommodated in the first accommodating part 24.

As shown in Fig. 3A, Fig. 3B, and Fig. 4, the first amplifying liquid holding part 40 holds the first amplifying liquid 41. The first amplifying liquid holding part 40 is constituted of, for example, a container 42 formed of a resin material and having an opening on one surface, and a sheet member 43 that covers the opening of the container 42 and that is breakable. The container 42 is filled with the first amplifying liquid 41, and the opening of the container 42 is sealed by the sheet member 43. The first amplifying liquid holding part 40 is arranged in the first accommodating part 24 in a posture in which the sheet member 43 faces upward.

The pressing force applied from the first pressing operation part 11 is transmitted to the sheet member 43 of the first amplifying liquid holding part 40 via the end part of the assay strip 1 to break the sheet member 43. The sheet member 43 is broken to immerse the end part (more specifically, the liquid feeding pad 4 described later) of the assay strip 1 in the first amplifying liquid 41. In the first pressing operation part 11 of the present example, a protruding part 11b that abuts on the sheet member 43. The protruding part 11b has, for example, an elongated shape extending in the longitudinal direction in the width direction of the assay strip 1 and has a tip with a pointed shape toward the sheet member 43, such that the sheet member 43 is easily broken.

### (Multifunctional member)

In addition, as shown in Fig. 2, Fig. 3A, Fig. 3B, and Fig. 4, the cartridge 100 includes a multifunctional member 30 having a function of accommodating the second amplifying liquid holding part 45. The multifunctional member 30 is arranged on the other end part (end part of the left side on the paper surface in Fig. 4) side of the case main body 20 and above the assay strip 1. The multifunctional member 30 is a member in which the second accommodating part 32 and the flow channel forming part 35 are integrally formed.

The second accommodating part 32 is a part accommodating the second amplifying liquid holding part 45. The second accommodating part 32 has a box shape having an opened upper surface. As shown in Fig. 4, on the bottom of the second accommodating part 32, a protrusion 34 for breaking a sheet member 48, which is described below, of the second amplifying liquid holding part 45, and a supply opening 32A that allows to supply the second amplifying liquid 46 flowed out from the second amplifying liquid holding part 45, toward the carrier 2.

Furthermore, the flow channel forming part 35 is provided to be connected from the second accommodating part 32. The flow channel forming part 35 has a flat plate shape, is arranged at a position facing the assay region L1 or the like in the longitudinal direction of the assay strip 1, and is arranged with an interval from the assay strip 1. Then, between the flow channel forming part 35 and the assay strip 1, a flow channel for flowing the second amplifying liquid 46 flowed out from the second accommodating part 32 toward the assay region L1 or the like is formed. The flow channel forming part 35 is arranged between the observation window 18 and the assay region L 1 or the like of the assay strip 1. Therefore, the flow channel forming part 35 is formed of a transparent member and thus the assay region L1 and the like can be observed through the observation window 18.

### (Second amplifying liquid holding part)

The second amplifying liquid holding part 45 holds the second amplifying liquid 46. The second amplifying liquid holding part 45 is constituted of, for example, a container 47 formed of a resin material and having an opening on one surface, and a sheet member 48 that covers the opening of the container 47 and that is breakable. The container 47 is filled with the second amplifying liquid 46, and the opening of the container 47 is sealed by the sheet member 48. The second amplifying liquid holding part 45 is arranged in the second accommodating part 32 in a posture in which the sheet member 48 faces downward. Accordingly, the sheet member 48 faces the protrusion 34 in the second accommodating part 32.

The pressing force applied from the second pressing operation part 12 to the second amplifying liquid holding part 45 acts in a direction of pushing down the second amplifying liquid holding part 45 downwardly, whereby the sheet member 43 is pressed against the protrusion 34. The sheet member 48 is pressed against the protrusion 34 to break the sheet member 48. The sheet member 48 is broken, and thus the second amplifying liquid 46 flows out through the flow channel formed by the supply opening 32A at the bottom of the second accommodating part 32 and the flow channel forming part 35.

As shown in Fig. 4, a gap (a clearance) C corresponding to the flow channel for the second amplifying liquid 46 is formed between a back surface 36 of the flow channel forming part 35 of the multifunctional member 30 and the carrier 2 of the assay strip 1. The gap C is, for example, in the range of 0.3 mm to 1 mm. The second amplifying liquid 46 flows out from the supply opening 32A at the bottom of the second accommodating part 32 toward the carrier 2, and the second amplifying liquid 46 that has flowed out flows through the flow channel formed by the gap C and reaches at least above the assay region L1. A part of the second amplifying liquid 46 that has reached the assay region L1 infiltrates into the carrier 2 from the flow channel, and a part thereof passes through the assay region L1. A part of the second amplifying liquid 46 that has passed through the assay region L1 is absorbed by the absorber 8 (see Fig. 2).

An absorption pad 6, which is described later, is arranged at one end part of the assay strip 1. In the case main body 20, a support part 22 that supports an end part of the assay strip 1 including the absorption pad 6 is formed at a position facing the absorption pad 6. A second accommodating part 32 of the multifunctional member 30 is arranged above the absorption pad 6. The support part 22 also supports the multifunctional member 30 via the absorption pad 6. In addition, in the case main body 20, a support part 21 that supports a central part of the assay strip 1 is formed.

### <Assay strip>

The assay strip 1 includes a carrier 2, a liquid feeding pad 4, and an absorption pad 6. Then, the carrier 2 is fixedly supported on a back pressure-sensitive adhesive sheet 7.

### (Carrier)

The carrier 2 is a porous insoluble carrier for developing a sample 50, and includes an assay region L1, a control region L2, and a color development region L3. In addition, the carrier 2 includes a label holding pad 3. The label holding pad 3 constitutes a spotting region on which the sample 50 is spotted from dropping port 16. The color development region L3 is arranged on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample 50 in the carrier 2.

It shows a state in which the assay region L1, the control region L2, and the color development region L3 are expressed as lines, but these are not always expressed. Details is described below, but before developing the sample 50 (see Fig. 5), the first amplifying liquid 41 (see Fig. 4), and the second amplifying liquid 46 (see Fig. 4), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by the increase of the color optical density in a case where the sample 50 is developed and the developed sample 50 is positive. Since the color development of the assay region L1 is amplified by silver amplification, which is described later, the assay region L1 develops a black color.

The control region L2 is also expressed as a line by increasing the color optical density in a case where the sample 50 is developed. Accordingly, the control region L2 becomes visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first amplifying liquid 41 is developed. However, the color development region L3 is expressed as an orange color line by changing a dark green color to an orange color in a case where the first amplifying liquid 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

### (Carrier - label holding pad)

As shown in Fig. 5, a labeling substance 53 is fixed to the label holding pad 3. The labeling substance 53 is modified with the first binding substance 52 that specifically binds to the test substance 51 contained in the sample 50. The label holding pad 3 is fixed on the carrier 2 at a position facing the dropping port 16 (see Fig. 2) of the cover member 10. Accordingly, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

### (Carrier - assay region)

As shown in Fig. 5, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bonded to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51.

### (Carrier - control region)

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

### (Carrier - color development region)

The color development region L3 contains a substance whose color development state changes by reacting with the first amplifying liquid 41. The color development region L3 shows that the first amplifying liquid 41 has been developed to that region by reacting with the first amplifying liquid 41 to develop a color or change a color. For example, in a case where a mixed aqueous solution of an iron nitrate aqueous solution and citric acid (manufactured by Fujifilm Wako Pure Chemical Corporation, 038-06925) is used as the first amplifying liquid 41, an aspect in which the color development region L3 is constituted of a color reagent immobilization line on which Bromocresol Green (manufactured by FUJIFELM Wako Pure Chemical Corporation) has been immobilized in a line shape is preferable. This aspect is the aspect of the color development region L3 of the present example. As described above, the color development region L3 of the present example is dark green color before reacting with the first amplifying liquid 41, and the dark green color is changed to an orange color in a case where the first amplifying liquid 41 reaches the color development region L3. The color development region L3 is sometimes referred to as an amplification index region because the timing of supplying the second amplifying liquid 46 after the first amplifying liquid 41 is developed is shown by changing the color development state.

### (Binding substance)

The first binding substance 52 that modifies the labeling substance 53 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like.

The second binding substance 56 that is immobilized in the assay region L1 and specifically binds to the test substance 51 is a substance that specifically binds to the test substance, for example, in a case where the test substance is an antigen, an antibody against the antigen, in a case where the test substance is an antibody, an antigen against the antibody, in a case where the test substance is a protein or a low-molecular-weight compound, an aptamer against the protein or the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an A type influenza virus or a biomarker thereof, anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

### (Liquid feeding pad)

The liquid feeding pad 4 is arranged in a state of being contact with one end of the carrier 2 and in the flow direction of the sample from the spotting region toward the assay region L1, which is constituted of the label holding pad 3, the first amplifying liquid 41 is fed to the carrier 2 from the upstream side of the spotting region. In the liquid feeding pad 4, in a case where the first pressing operation part 11 is pressed, one end of the liquid feeding pad 4 is immersed in the first amplifying liquid holding part 40. The liquid feeding pad 4 is formed of a porous material and absorbs the first amplifying liquid 41, and the absorbed first amplifying liquid 41 is fed to the carrier 2 by a capillary action.

### (Absorption pad)

The absorption pad 6 is arranged in a state of being contact with the other end of the carrier 2 and absorbs the sample 50 and the first amplifying liquid 41, which are developed on at least the carrier 2. As shown in Fig. 4, the absorption pad 6 is arranged on the upstream side of the supplying portion of the second amplifying liquid 46 in the flow direction of the second amplifying liquid 46. In the present example, the flow direction of the second amplifying liquid 46 is a direction from the supplying portion of the second amplifying liquid 46 toward the assay region L1, and is a direction opposite to the flow direction in which sample 50 spotted on the label holding pad 3 flows toward the assay region L1. The absorption pad 6 absorbs a sample 50 which is developed in a direction opposite to a flow direction of the second amplifying liquid 46 and which passes through the assay region L1. In addition, the absorption pad 6 absorbs the first amplifying liquid 41 that is fed to the carrier 2 via the liquid feeding pad 4.

The absorption pad 6 is formed of a porous material and absorbs the sample 50 and the first amplifying liquid 41 from the carrier 2 by a capillary force. The absorption pad 6 absorbs the sample 50 and the first amplifying liquid 41 at the end part of the carrier 2, thereby the sample 50 and the first amplifying liquid 41 is suppressed from staying in the carrier 2, and the sample 50 and the first amplifying liquid 41 can be smoothly flowed toward the absorption pad 6. As a result, the sample 50 and the first amplifying liquid 41 can be smoothly flowed from the label holding pad 3, which is a spotting region of the sample 50, toward the assay region L1.

The absorption pad 6 is provided, for example, also in the assay cartridge described in the related art. It is the above-described advantage that the sample 50 and the first amplifying liquid 41 can be smoothly flowed in the carrier 2, and the absorption pad 6 is thus often provided in an assay cartridge in the related art. The absorption pad 6 is also provided in the assay cartridge 100 of the present example, but this is not an essential configuration in the assay cartridge of the present disclosure, and the absorption pad 6 may not necessarily be provided. It is conceived that for example, in a case where the size of the carrier 2 such as the length and the width of the carrier 2 in the assay strip 1 is large, the absorption amounts of the sample 50 and the first amplifying liquid 41 are also increased and the retention is also suppressed. In this manner, in a case where the spreading force that makes the sample 50 and the first amplifying liquid 41 flow smoothly to the assay region L1 can be exhibited, a configuration in which the absorption pad 6 is not provided is also possible.

### <Absorber>

An absorber 8 absorbs at least a part of the second amplifying liquid 46 which is supplied from the second accommodating part 32, flows on the surface of the assay strip 1 toward the assay region L1, and passes through the assay region L1. The absorber 8 and the absorption pad 6 are the same in that they have a function of absorbing a liquid. However, the role of both is different as described above. That is, the absorber 8 has a role of absorbing at least a part of the second amplifying liquid 46 that has passed through the assay region L1, whereas the absorption pad 6 has a role of absorbing the sample 50 and the first amplifying liquid 41. A part of the second amplifying liquid 46 that has flowed out from the second accommodating part 32 toward the assay region L1 may flow back and reache the absorption pad 6, and in that case, the absorption pad 6 absorbs the second amplifying liquid 46 that has flowed back.

In addition, the arrangement position of the absorber 8 and the arrangement position of the absorption pad 6 are also different from each other. As illustrated in Fig. 4, in the longitudinal direction of the assay strip 1, the absorber 8 is arranged on the downstream side of the second amplifying liquid 46 in the flow direction from the second accommodating part 32 toward the assay region L, and more specifically, in the present example, the absorber 8 is arranged between the assay region L1 and the label holding pad 3 which is a spotting region of the sample 50. On the other hand, the absorption pad 6 is arranged on the upstream side of the supplying portion of the second amplifying liquid 46 from the second accommodating part 32 in the flow direction of the second amplifying liquid 46, and more specifically, the absorption pad 6 in the assay strip 1 of the present example, is arranged at the end part on the side opposite to the liquid feeding pad 4. In a case where the absorber 8 is defined as a first absorber in the present disclosure, the absorption pad 6 is an example of a second absorber in the present disclosure.

The cartridge 100 of the present example is characterized that the absorber 8 is provided, but the detail of the absorber 8 is described after a basic configuration, a basic method for using, and the like of the cartridge 100 is described.

### <Amplifying liquid>

In the present embodiment, the amplifying liquid is a two-component type amplifying liquid including the first amplifying liquid 41 and the second amplifying liquid 46. The color development in the assay region L1 and the control region L2 is amplified by reacting the first amplifying liquid 41 with the second amplifying liquid 46 on the assay strip 1. In a case where a metal-based labeling substance such as a gold colloid is used as the labeling substance 53 as in the present example, for example, silver amplification is used as a method of amplifying the labeling signal of the labeling substance 53. The first amplifying liquid 41 and the second amplifying liquid 46 are, as an example, amplifying liquids used for silver amplification, and the reaction between the first amplifying liquid 41 and the second amplifying liquid 46 using the labeling substance 53 as a catalyst is an amplification reaction. By the amplification reaction, silver particles having a particle diameter relatively larger than that of the labeling substance 53 are generated.

More specifically, in the present example, the first amplifying liquid 41 is a reducing agent that reduces silver ions, and the second amplifying liquid 46 is a silver ion. In a case where the first amplifying liquid 41, which is a reducing agent, and the second amplifying liquid 46 containing a silver ion, are brought into contact with the labeling substance 53, silver particles (see Fig. 5) are generated, and the generated silver particles deposits on the labeling substance 53 using the labeling substance 53 as a nucleus. By depositing the silver particles on the labeling substance 53, silver particles 60 having a particle diameter larger than that of the labeling substance 53 (see Fig. 5) are generated. Accordingly, the labeling signal issued by the labeling substance 53 is amplified, and as a result, the color development of the labeling substance 53 is amplified in the assay region L1 and the control region L2.

### (First amplifying liquid)

As the reducing agent as the first amplifying liquid 41, any inorganic or organic material or a mixture thereof can be used as long as the silver ion used as the second amplifying liquid 46 can be reduced to silver. Preferred examples of the inorganic reducing agent include a reducing metal salt and a reducing metal complex salt, of which the atomic valence is capable of being changed with a metal ion such as Fe²⁺, V²⁺, or Ti³⁺. In a case where an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of Fe²⁺ is preferably used.

It is also possible to use a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

### (Second amplifying liquid)

The solution containing silver ions, which is used as the second amplifying liquid 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

### <Immunochromatographic method>

An immunochromatographic method will be described with reference to Fig. 5. Here, a case where the sample 50 includes the test substance 51, that is, on the premise that the sample 50 is positive is described.

First, the sample 50 is spotted on the label holding pad 3 which is the spotting region (Step S1). The test substance 51 in the sample 50 spotted on the label holding pad 3, specifically binds to the first binding substance 52 that modifies the labeling substance 53 contained in the label holding pad 3. By the capillary action in the carrier 2, in the carrier 2, the sample 50 is developed from the label holding pad 3 toward the assay region L1. A part of the sample 50 is also developed in a direction opposite to the assay region L1.

Next, the first amplifying liquid 41 is supplied (Step S2). The first amplifying liquid 41 is supplied from the liquid feeding pad 4 side. The first amplifying liquid 41 is supplied to the carrier 2 through the liquid feeding pad 4 and is developed toward the assay region L1.

After that, the process waits until the first amplifying liquid 41 is developed in the assay region L1 (Step S3 and Step S4). "Wait" shown in Fig. 5 means an action of waiting. The first amplifying liquid 41 is gradually developed toward the assay region L1, and the sample 50 to be developed from the label holding pad 3 and the labeling substance 53 modified with the first binding substance 52 are developed toward the assay region L1 to be pushed by the first amplifying liquid 41 (Step S3).

The test substance 51 in the sample 50 that has reached the assay region L1 is captured by the second binding substance 56 in the assay region L1. That is, the labeling substance 53 bound via the test substance 51 and the first binding substance 52 is captured in the assay region L1. On the other hand, the labeling substance 53 that is not bound to the test substance 51 passes through the assay region L1 without being captured and is captured by the third binding substance 58 in the control region L2.

In a case where the development of the first amplifying liquid 41 proceeds and the first amplifying liquid 41 reaches the color development region L3 (Step S4), the color development region L3 reacts with the first amplifying liquid 41 to change the color development state. In the present example, the color development region L3 is dark green color before reacting with the first amplifying liquid 41, and the dark green color is changed to an orange color by reacting with the first amplifying liquid 41.

After the first amplifying liquid 41 is sufficiently developed, the second amplifying liquid 46 is supplied to the carrier 2 (Step S5). The second amplifying liquid 46 is supplied to the carrier 2 from the absorption pad 6 side of the color development region L3 and is developed toward the assay region L 1. Here, the first amplifying liquid 41 is a first amplifying liquid containing a reducing agent that reduces silver ions, and the second amplifying liquid 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

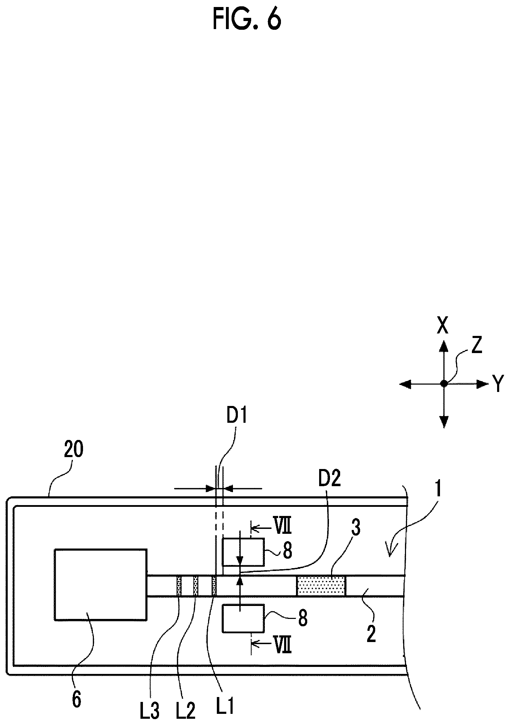

The arrangement of the absorber 8 in the cartridge 100 is described with reference to Fig. 6 and below.

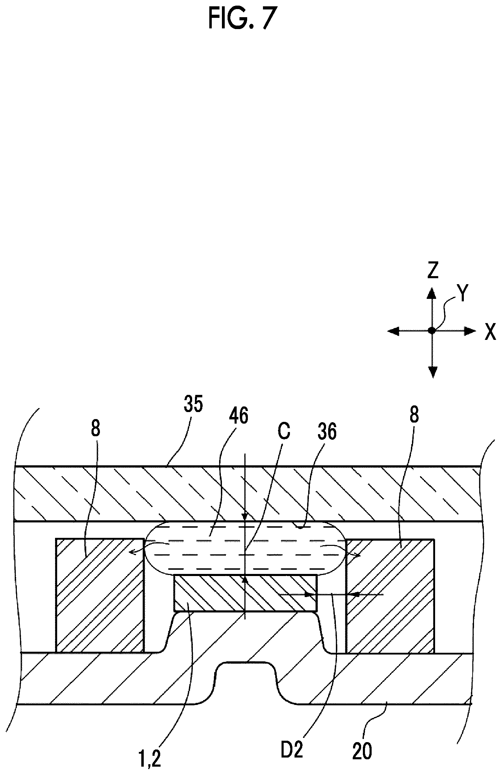

Fig. 6 is an enlarged view of a part of the cartridge 100 showing an arrangement of the absorber 8 in the cartridge 100 shown in Fig. 1 to Fig. 4, and Fig. 7 is a cross-sectional view taken along a line VII-VII in Fig. 6.

As shown in Fig. 6, an absorber 8 absorbs at least a part of the second amplifying liquid 46, which is one of the amplifying liquids and which is supplied from the second accommodating part 32, flows on the surface of the assay strip 1 toward the assay region L1, and passed through the assay region L1. In the flow direction of the second amplifying liquid 46, the assay region L1 is arranged on the downstream side of a supplying portion of the second amplifying liquid 46, and the spotting region is arranged on the further downstream side of the assay region L1. In the present embodiment, the absorber 8 is arranged on the downstream side of the assay region L1 in the flow direction (see Fig. 4) of the second amplifying liquid 46. In addition, the absorber 8 is arranged not to overlap with the assay region L1 in the flow direction of the second amplifying liquid 46. That is, in the longitudinal direction (the Y direction in the figure) of the assay strip 1, the absorber 8 is arranged between the assay region L1 and the label holding pad 3 corresponding to the spotting region (see also Fig. 2 and Fig. 4). The absorber 8 is arranged on both sides of the assay strip 1 in the width direction, and the absorber 8 is arranged with an interval D1 from the assay strip 1.

In the cartridge 100 according to the present embodiment, the first amplifying liquid 41 is developed toward the assay region L1 because of the capillary action in the liquid feeding pad 4 and the carrier 2. On the other hand, the second amplifying liquid 46 flows on the surface of the assay strip 1 using the gap C between the flow channel forming part 35 of the multifunctional member 30 and the surface of the carrier 2 as a flow channel and is developed in the assay region L1. In a case where the second amplifying liquid 46 flows out from the supply opening 32A at the bottom of the second accommodating part 32 of the multifunctional member 30 into the gap C serving as a flow channel and in a case where the second amplifying liquid 46 flows through the gap C, bubbles may be generated in the gap C. That is, in a case where the second amplifying liquid 46 is supplied to the carrier 2, bubbles may be generated on the assay region L1 arranged at a position facing the flow channel forming part 35 of the carrier 2 and the periphery of the assay region L1. It is presumed that one of the causes of the generation of bubbles is that the second amplifying liquid 46 flows vigorously into the gap C and the accumulated air and the second amplifying liquid 46 are mixed with each other. In addition, it is also presumed that the cause is that, in a case where the second amplifying liquid 46 that has flowed into the gap C infiltrates into the carrier 2, the air in the carrier 2 floats up on the surface of the carrier 2. It is presumed that bubbles are generated because of at least one of these.

The bubbles generated in the second amplifying liquid 46 become noise in a case where the color development state of the assay region L1 is determined. Therefore, in order to make an accurate determination in the assay, it is necessary to wait for the disappearance of the bubbles.

In a cartridge not including the absorber 8 in the related art, the most part of the second amplifying liquid 46 that has passed through the assay region L1, which has not infiltrated into the carrier 2, flows back to the absorption pad 6 side, and finally is absorbed to the absorption pad 6. The second amplifying liquid 46 is absorbed by the carrier 2 or the absorption pad 6 and discharged from the flow channel (the gap C), thereby the bubbles on the assay region L1 also disappear. However, since the second amplifying liquid 46 that has passed through the assay region L1 flows back to the absorption pad 6 side, it is necessary to wait for the retreat of the second amplifying liquid 46 from the assay region L1. Therefore, it takes time until the bubbles disappear. This time is rate-determining, and thus the improvement of the throughput of the assay has been difficult.

On the other hand, the cartridge 100 of the present disclosure includes the absorber 8 that absorbs at least a part of the second amplifying liquid 46 which flows on the surface of the assay strip 1 toward the assay region L1, and passes through the assay region L1. Therefore, in the cartridge 100 of the present disclosure, as compared with the cartridge in the related art which does not include the absorber 8, the second amplifying liquid 46 can be quickly retreated from the assay region L1 because of the absorption action of the absorber 8. That is, the second amplifying liquid 46 supplied from the second accommodating part 32 passes through the assay region L1 once to the downstream side (see Fig. 4), and then a part of the second amplifying liquid 46 may flow back toward the assay region L1. In that case, the bubbles caused by the second amplifying liquid 46 remain in the assay region L1 until the second amplifying liquid 46 that has flowed back is retreated from the assay region L1. In the cartridge 100 of the present disclosure, since the absorber 8 absorbs at least a part of the second amplifying liquid 46 that has once passed through the assay region L1, the back flow rate at which the second amplifying liquid 46 flows back toward the assay region L1 is reduced. Therefore, the second amplifying liquid 46 can be quickly retracted from the assay region L1 as compared to the related art. Accordingly, it is possible to shorten a time until the bubbles disappear, and the throughput of the assay can be improved.

The "disappearance" of bubbles means that at least a part of the bubbles generated in the amplifying liquid is made disappear. The "time until the disappearance" refers to, in addition to the time taken for the bubbles in the amplifying liquid to completely disappear, a time until the amount of the bubbles, which becomes noise with respect to the signal indicating the color development state of the assay region in a case where a part of the bubbles disappear, is reduced to a range that does not affect the assay accuracy.

As described above, in the cartridge 100 according to the present embodiment, the absorber 8 is arranged on a downstream side of the assay region L1 in a flow direction of the amplifying liquid 46, and the absorber 8 is not overlapped with the assay region L1 in the flow direction of the second amplifying liquid 46. Because of such arrangement, only the second amplifying liquid 46 that has passed through the assay region L1 can be absorbed by the absorber 8, and it is possible to suppress the absorption of the second amplifying liquid 46, which affects an amplification action of the color development with respect to the assay region L1, by the absorber 8. That is, it is possible to prevent the decrease in the effect of amplifying the color development by the second amplifying liquid 46 with respect to the assay region L 1.

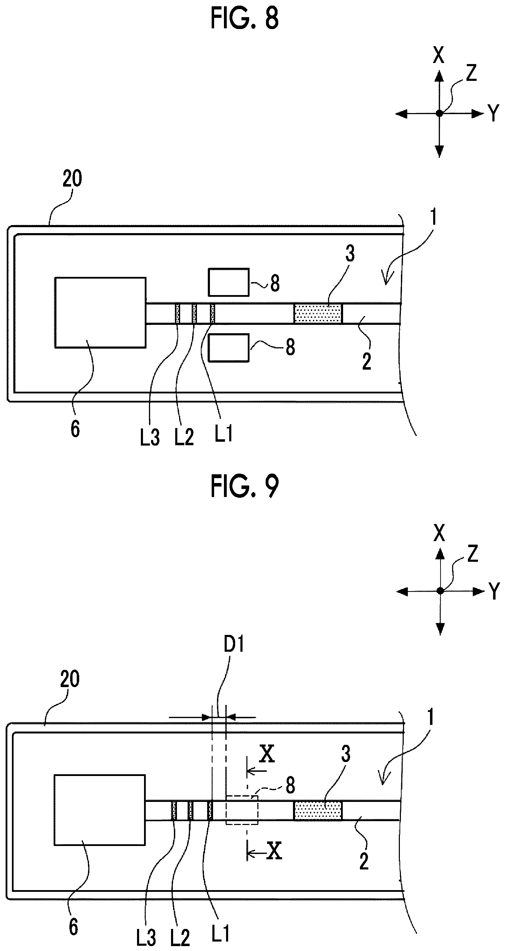

As shown in Fig. 8, the absorber 8 may be arranged to overlap with the assay region L1 in the flow direction of the second amplifying liquid 46. In a case where the amount of the second amplifying liquid 46 flowing into the assay region L1 is large, the effect of amplifying the color development can be ensured by the short-time contact between the second amplifying liquid 46 and the assay region L1. In that case, the absorption of the second amplifying liquid 46 on the assay region L1 may be carried out in a short time to shorten the disappearance time of bubbles.

The description that the absorber 8 and the assay region L1 "do not overlap in the flow direction" means that an interval D1 between the end part of the assay region L1 shown in Fig. 6 on the absorber 8 side and the end part of the absorber 8 on the assay region L1 side is larger than 0 (D1 > 0).

In the cartridge 100 according to the present embodiment, the absorber 8 is arranged on both sides in the width direction of the assay strip 1, but it may be arranged on at least one of both sides. However, in a case where the absorber 8 is arranged on both sides of the assay strip 1 in the width direction, the second amplifying liquid 46 can be absorbed more quickly as compared with the case where the absorber 8 is arranged on only one side. Therefore, the effect of shortening the disappearance time of the bubbles is high.

In the cartridge 100 according to the present embodiment, the absorber 8 is arranged with an interval D2 from the assay strip 1. In the cartridge 100 according to the present embodiment, the flow direction of the second amplifying liquid 46 and the flow direction of the sample 50 are opposite to each other, and the absorber 8 is arranged between the label holding pad 3 which is a spotting region of the sample 50, and the assay region L1. Therefore, in a case where the sample 50 is developed from the label holding pad 3 toward the assay region L1, and a part of the sample 50 is absorbed by the absorber 8 in a case where the absorber 8 is in contact with the assay strip 1, and the amount of the sample 50 developed to the assay region L1 may be reduced. However, in the cartridge 100 according to the present embodiment, since the absorber 8 is arranged with the interval D2 from the assay strip 1, the sample 50 can be developed in the assay region L1 without being absorbed by the absorber 8.

The second amplifying liquid 46 flows through the gap C between the surface of the assay strip 1 and the back surface 36 of the flow channel forming part 35 as a flow channel. At this time, the second amplifying liquid 46 sandwiched between a surface of the assay strip 1 and the flow channel forming part 35 flows in a state where a portion that protrudes from the width of the assay strip 1 because of surface tension in the width direction of the assay strip 1 (see Fig. 6). Therefore, as described above, even in a case where the absorber 8 is arranged with the interval D2 from the assay strips 1, the second amplifying liquid 46 flowing through the gap C can be absorbed. The interval D2 between the absorber 8 and the assay strip 1 is preferably 0.3 mm or more and 1 mm or less and more preferably 0.4 mm or more and 1 mm or less. In a case of setting the interval D2 to 0.3 mm or more, it is possible to suppress the contact between the absorber 8 and the assay strip 1 in a case where the external force of the cartridge 100 is applied, or the like. In addition, in a case where the interval D2 is too large, the absorbency of the second amplifying liquid 46 by the absorber 8 is reduced. Therefore, in a case where the interval D2 is set to 1 mm or less, the absorbency of the second amplifying liquid 46 can be ensured.

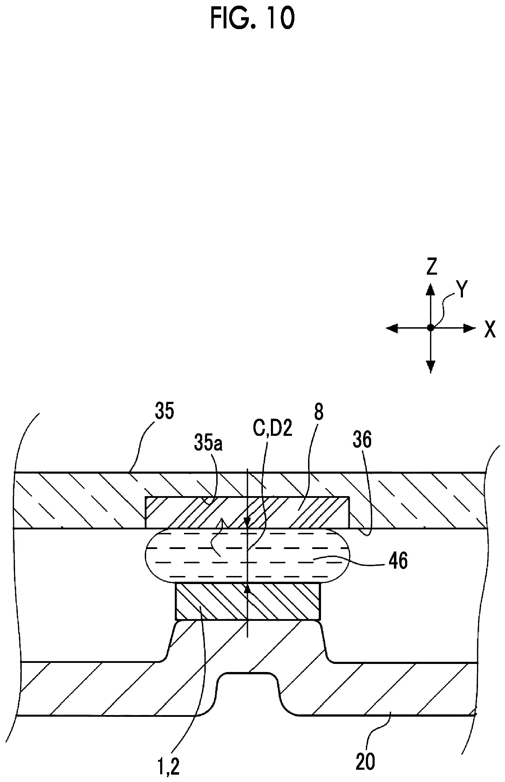

In the cartridge 100 of the present disclosure, as shown in Fig. 2, Fig. 6, and Fig. 7, the absorber 8 is arranged on both sides of the assay strip 1 in the width direction, but the embodiment of the present disclosure is not limited thereto. For example, as shown in Fig. 9 and Fig. 10, the absorber 8 may be arranged at a position facing the surface of the assay strip 1. Fig. 9 is a plan view showing a positional relationship between the assay strip 1 and the absorber 8 in a case where the absorber 8 is arranged at a position facing the surface of the assay strip 1, and Fig. 10 is a cross-sectional view taken along line X-X of Fig. 9.

In an example shown in Fig. 9 and Fig. 10, at least a part of the absorber 8 is arranged at a position facing the surface of the assay strip 1. In the modification example shown in Fig. 9 and Fig. 10, the absorber 8 is accommodated in a recess 35a provided on the surface (the back surface 36 of the flow channel forming member) of the flow channel forming part 35 facing the assay strip 1. In the present example, the absorber 8 is accommodated in the recess 35a, and the back surface 36 of the flow channel forming part 35 and the surface of the absorber 8 are flush with each other. In the present example, the interval D2 between the absorber 8 and the assay strip 1 is a distance between the surface of the assay strip 1 (the surface of the carrier 2) and the surface (the back surface 36) of the absorber 8 facing the assay strip 1, and the interval D2 has the same distance as that of the gap C. Here, the interval D2 is preferably 0.3 mm or more and 1 mm or less and more preferably 0.4 mm or more and 1 mm or less. The surface of the absorber 8 may protrude or may be recessed from the back surface 36 of the flow channel forming part 35.

As shown in Fig. 9 and Fig. 10, also in a case where at least a part of the absorber 8 is arranged at a position facing the surface of the assay strip 1, the same effect is exhibited as the case where the absorber 8 is arranged in the width direction of the assay strip 1. That is, since the absorber 8 that absorbs at least a part of the second amplifying liquid 46 that has flowed toward the assay region L1 on the surface of the assay strip 1 and has passed through the assay region L1 is provided, it is possible to discharge more quickly the second amplifying liquid 46 that has passed through the assay region L1, from the passage by the absorber 8 as compared with a case where the absorber 8 is not provided. Accordingly, it is possible to shorten a time until the bubbles disappear, and the throughput of the assay can be improved.

The amplifying liquid applied in the assay cartridge 100 of the present disclosure is not limited to the above-described two-component type amplifying liquid, and may be an amplifying liquid that can amplify the color development in the assay region L1 with one component. Also in the amplifying liquid with one component, in a case of including the absorber 8 that absorbs at least a part of the amplifying liquid which flows on the surface of the assay strip 1 toward the assay region L1, and passed through the assay region L1, the same effect can be obtained.
1: assay strip
2: immunochromatographic carrier (carrier)
3: label holding pad
4: liquid feeding pad
6: absorption pad (second absorber)
7: back pressure-sensitive adhesive sheet
8: absorber (first absorber)
9: case
10: cover member
11: first pressing operation part
11b: protruding part
12: second pressing operation part
12b: abutting part
16: dropping port
18: observation window
20: case main body
21: support part
22: support part
24: first accommodating part
30: multifunctional member
32: second accommodating part
32A: supply opening
34: protrusion
35: flow channel forming part
35a: recess
36: back surface
40: first amplifying liquid holding part
41: first amplifying liquid
42: container
43: sheet member
45: second amplifying liquid holding part
46: second amplifying liquid
50: sample
51: test substance
52: first binding substance
53: labeling substance
56: second binding substance
58: third binding substance
60: silver particles
100: cartridge (assay cartridge)
L1: assay region
L2: control region
L3: color development region

## Claims

1. An assay cartridge that is used for immunochromatographic assay, the assay cartridge comprising:
an assay strip that has, on a surface, an assay region in which a color development state changes depending on whether a sample is positive or negative;
an accommodating part that accommodates an amplifying liquid which amplifies color development of the assay region; and
an absorber that absorbs at least a part of the amplifying liquid which is supplied from the accommodating part, flows on the surface of the assay strip toward the assay region, and passes through the assay region.

2. The assay cartridge according to claim 1,
wherein the absorber is arranged on a downstream side of the assay region in a flow direction of the amplifying liquid, and the absorber is not overlapped with the assay region in the flow direction.

3. The assay cartridge according to claim 1 or 2,
wherein in a case where a direction intersecting the flow direction of the amplifying liquid is defined as a width direction,
the absorber is arranged on at least one of both sides of the assay strip in the width direction.

4. The assay cartridge according to claim 3,
wherein the absorber is arranged on both sides of the assay strip in the width direction.

5. The assay cartridge according to claim 1 or 2,
wherein at least a part of the absorber is arranged at a position facing the surface of the assay strip.

6. The assay cartridge according to any one of claims 1 to 5,
wherein the absorber is arranged with an interval from the assay strip.

7. The assay cartridge according to claim 6,
wherein the interval between the absorber and the assay strip is 0.3 mm or more and 1 mm or less.

8. The assay cartridge according to any one of claims 1 to 7,
wherein in the assay strip, a spotting region on which the sample is spotted is arranged on a downstream side of the assay region in a flow direction of the amplifying liquid.

9. The assay cartridge according to any one of claims 1 to 8,
wherein the assay strip includes a second absorber that absorbs the sample which is developed in a direction opposite to a flow direction of the amplifying liquid and which passes through the assay region, that is arranged on an upstream side of a supplying portion of the amplifying liquid from the accommodating part, and that is different from the absorber.
